# EUROPÄISCHE PATENTSCHRIFT

(11) **EP 3 680 320 B1**
(45) Veröffentlichungstag und Bekanntmachung des Hinweises auf die Patenterteilung: **04.08.2021**
(21) Anmeldenummer: 19214447.5
(22) Anmeldetag: 09.12.2019
(51) Int. Cl.: C12M 1/00, C12M 3/00

(54) **AUSTRAGSVORRICHTUNG FÜR EIN BESCHICKUNGSSYSTEM ZUM BEREITSTELLEN VON BIOMASSE IN EINER BIOGASANLAGE**
DELIVERY DEVICE FOR A CHARGING SYSTEM FOR PROVIDING BIOMASS IN A BIOGAS SYSTEM
DISPOSITIF DE DÉCHARGE POUR UN SYSTÈME DE CHARGEMENT PERMETTANT DE FOURNIR DE LA BIOMASSE DANS UNE INSTALLATION DE BIOGAZ

(30) Priorität: 09.01.2019 DE 102019100407
(43) Veröffentlichungstag der Anmeldung: 15.07.2020
(73) Patentinhaber: Fliegl Agrartechnik GmbH, 84453 Mühldorf am Inn (DE)
(72) Erfinder: Fliegl jun., Josef, 84453 Mühldorf (DE)
(74) Vertreter: Nußbaum, Christopher

(56) Entgegenhaltungen:
- EP-A1- 0 529 113
- EP-A1- 1 681 344
- DE-A1-102011 088 319
- DE-U- 1 891 383
- DE-U1- 20 008 186

## Beschreibung

Die Erfindung betrifft eine Austragsvorrichtung für ein Beschickungssystem zum Bereitstellen von Biomasse in einer Biogasanlage. Des Weiteren betrifft die Erfindung noch ein Beschickungssystem zum Beschicken einer Biogasanlage mit Biomasse. Biogasanlagen dienen zur Erzeugung von Biogas durch Vergärung von Biomasse. In landwirtschaftlichen Biogasanlagen werden meist tierische Exkremente, wie z.B. Gülle oder Festmist, und Energiepflanzen als Substrat eingesetzt. In nicht-landwirtschaftlichen Anlagen wird Material aus der Biotonne verwendet oder Abfallprodukte aus der Lebensmittelproduktion.

Zum Beschicken, also Versorgen von Biogasanlagen mit Biomasse, werden unterschiedlichste Beschickungssysteme verwendet. Durchaus gängig sind beispielsweise Beschickungssysteme mit einem flüssigkeitsdichten Behälter, der im Inneren eine um eine Rotationsachse drehbare Austragsvorrichtung - auch als Austragsschwert bezeichnet - aufweist, mittels welcher in dem Behälter enthaltene Biomasse zu einer Austragsöffnung befördert werden kann, von wo aus diese beispielsweise mittels einer Förderschnecke zur betreffenden Biogasanlage weiterbefördert werden kann.

Je nach eingesetzter Biomasse und Befüllgrad eines Behälters eines solchen Beschickungssystems kann die aufzubringende Kraft durch eine Austragsvorrichtung variieren. Wenn man verhindern möchte, dass die Austragsvorrichtung ins Stocken gerät, muss eine Antriebseinrichtung, welche zum Antreiben der Austragsvorrichtung dient, entsprechend groß dimensioniert werden. Wenn solch eine Austragsvorrichtung während der Rotationsbewegung auf zu große Widerstände stößt, beispielsweise durch große Brocken in der Biomasse, kommt es zu hohen Belastungen an der Antriebseinrichtung, die zum Antreiben der Austragsvorrichtung dient. So können beispielsweise Schäden in einem Getriebe entstehen oder es kommt zu einer Abschaltung eines Motors, mittels welchem die Austragsvorrichtung angetrieben wird. Üblicherweise sind Behälter solcher Beschickungssysteme, in denen derartige Austragsvorrichtungen eingesetzt werden, auch so geformt, dass die Biomasse nicht seitlich bzw. nach radial außen ausweichen kann, wenn die Austragsvorrichtung auf die Biomasse trifft. Dadurch können auch relativ große Kräfte auf die Austragsvorrichtung und somit auf die zugehörige Antriebseinrichtung einwirken, welche zum Antreiben der Austragsvorrichtung dient.

Zudem ist es an sich wünschenswert, eine möglichst hohe Austragsleistung an Biomasse zu erzielen. Eine wesentliche Herausforderung bei der Verwendung solcher Austragsvorrichtungen besteht somit darin, eine möglich hohe Austragsleistung hinsichtlich der Biomasse bei gleichzeitig möglichst schonendem Betrieb einer Antriebseinrichtung zu erzielen, mittels welcher die Austragsvorrichtung angetrieben wird.

Die EP 0 529 113 A1 zeigt eine Silo-Austragsvorrichtung mit einem in einem Silo befindlichen Rotor, an dem mindestens ein Räumarm in tangentialer Richtung befestigt ist, der ein Blattfederpaket aufweist. An einem äußeren Ende des Räumarms ist ein Räumwerkzeug befestigt.

Die DE 200 08 186 U1 zeigt eine Austragseinrichtung für einen Fermenter. An einer Räumwerkwelle sind zwei Räumflügel gelenkig und federnd befestigt, an denen jeweilige Räumer angebracht sind.

Es ist daher die Aufgabe der vorliegenden Erfindung, eine Austragsvorrichtung bereitzustellen, mittels welcher Biomasse mit einer möglichst hohen Austragsleistung befördert werden kann, wobei gleichzeitig eine zum Antreiben der Austragsvorrichtung dienende Antriebseinrichtung möglichst schonend betrieben werden kann.

Diese Aufgabe wird durch eine Austragsvorrichtung für ein Beschickungssystem, das zum Beschicken einer Biogasanlage mit Biomasse ausgelegt ist, mit den Merkmalen des Patentanspruchs 1 gelöst. Vorteilhafte Ausgestaltungen mit zweckmäßigen und nicht trivialen Weiterbildungen der Erfindung sind in den abhängigen Ansprüchen angegeben.

Die erfindungsgemäße Austragsvorrichtung für ein Beschickungssystem, das zum Beschicken einer Biogasanlage mit Biomasse ausgelegt ist, umfasst einen Anschlusskörper mit einer Schnittstelle für eine Antriebseinrichtung des Beschickungssystems, mittels welcher die Austragsvorrichtung um eine Rotationsachse in Rotation versetzbar ist. Bei der Schnittstelle des Anschlusskörpers kann es sich beispielsweise um eine Nabe, einen Flansch oder dergleichen handeln, mittels welcher der Anschlusskörper zum Beispiel mit einem Getriebe, welches Teil der besagten Antriebseinrichtung des Beschickungssystems sein kann, verbunden werden kann. Bei dem Getriebe kann es sich beispielsweise um ein Planetengetriebe handeln, welches über eine Antriebswelle oder dergleichen mit einem Motor, beispielsweise einem Elektromotor, verbunden werden kann. In dem beschriebenen Fall bilden das Planetengetriebe, die Antriebswelle und der Elektromotor die besagte Antriebseinrichtung des Beschickungssystems, mittels welcher die Austragsvorrichtung um die besagte Rotationsachse in Rotation versetzbar ist.

Die erfindungsgemäße Austragsvorrichtung umfasst zudem zumindest einen sich in radialer Richtung von dem Anschlusskörper nach außen erstreckenden Austragsarm zum Befördern der Biomasse zu einer Austragsöffnung des Beschickungssystems, wobei der Austragsarm einen starr mit dem Anschlusskörper verbundenen Hauptarm und einen im Bereich eines vom Anschlusskörper abgewandten Längsende des Hauptarms angeordneten Armfortsatz aufweist, der um eine parallel zur Rotationsachse verlaufende Drehachse verdrehbar am Hauptarm gelagert ist. Zudem weist die erfindungsgemäße Austragsvorrichtung ein Rückstellelement auf, welches ein Rückstellmoment auf den Armfortsatz in Richtung einer Neutralstellung ausübt, in welcher jeweilige Längsachsen des Hauptarms und des Armfortsatzes parallel zueinander ausgerichtet sind.

Wird also die Austragsvorrichtung mittels der Antriebseinrichtung um die besagte Rotationsachse in Rotation versetzt, wird der zumindest eine sich radial nach außen erstreckende Austragsarm um die Rotationsachse verdreht. In einem Behälter des Beschickungssystems angeordnete Biomasse kann so mittels der ebenfalls im Behälter des Beschickungssystems angeordneten Austragsvorrichtung zur besagten Austragsöffnung des Beschickungssystems befördert werden. Dadurch, dass der Hauptarm des Austragsarms starr mit dem Anschlusskörper verbunden ist, folgt dieser also immer der Rotationsbewegung des Anschlusskörpers, welcher mittels der Antriebseinrichtung angetrieben werden kann.

Steigt nun das auf den Armfortsatz ausgeübte Moment so sehr an, dass es das Rückstellmoment, welches mittels des Rückstellelements ausgeübt wird, übersteigt, so knickt der am Hauptarm angeordnete Armfortsatz um die Drehachse weg. Mit anderen Worten kann also der Armfortsatz um die Drehachse, welche sich parallel zur Rotationsachse der Austragsvorrichtung erstreckt, verdreht werden, wenn ein entsprechend hohes Moment bzw. eine entsprechend hohe Kraft auf den Armfortsatz ausgeübt wird. Dies kann beispielsweise der Fall sein, wenn der Armfortsatz auf große Materialbrocken in der Biomasse trifft. Durch das Wegschwenken des Armfortsatzes wird verhindert, dass ein zu starkes Gegenmoment auf die Antriebseinrichtung ausgeübt wird, mittels welcher die Austragsvorrichtung angetrieben wird. Vor allem ein Getriebe der Antriebseinrichtung kann dadurch geschont werden, das für einen besonders reibungslosen bzw. ausfallarmen Betrieb sorgen kann.

So beispielsweise ein Brocken, auf den der Armfortsatz getroffen ist, durch Wegschwenken des Armfortsatzes bei weiterer Drehbewegung der Austragsvorrichtung passiert werden. Danach kann der Armfortsatz - sofern die Biomasse keine zu große Gegenkraft ausübt - wieder in die Neutralstellung zurückschwenken, nämlich indem das Rückstellelement das entsprechende Rückstellmoment ausübt.

Neben der Zusammensetzung der Biomasse, insbesondere der Dichte, und dem Füllstand eines Behälters des Beschickungssystems mit der Biomasse bestimmt zudem maßgeblich die Länge des zumindest einen Austragsarms, was für ein Antriebsmoment aufgebracht werden muss, um die Austragsvorrichtung in Rotation zu versetzen und somit die im Beschickungssystem enthaltene Biomasse zur Austragsöffnung des Beschickungssystems zu befördern. Je weiter außen am Austragsarm eine Kraft in Umfangsrichtung auf den Austragsarm wirkt, desto größer ist das erforderliche Antriebsmoment, um den Austragsarm in Rotation zu versetzen bzw. eine Rotationsbewegung beizubehalten. Befindet sich also z.B. Biomasse entlang des gesamten Austragsarms, so muss ein größeres Antriebsmoment zur Rotation des Austragsarms aufgebracht werden als wenn nur entlang einer Teillänge des Austragsarms Biomasse angeordnet ist.

Dadurch, dass der am Hauptarm angeordnete Armfortsatz um die parallel zur Rotationsachse verlaufende Drehachse verdrehbar am Hauptarm gelagert ist, kann das erforderliche Moment, welches die Antriebseinrichtung des Beschickungssystems bereitstellen muss, um die Austragsvorrichtung um die Rotationsachse in Rotation zu versetzen, auf ein gewisses Maß beschränkt werden. Denn hat sich entlang der Haupterstreckungsrichtung des Austragsarms entsprechend viel Biomasse angesammelt, welche durch entsprechende Rotation der Austragsvorrichtung bewegt werden soll, so kann der drehbar am Hauptarm angeordnete Armfortsatz wegknicken, in Folge dessen das aufzubringende Moment zum Antreiben der Austragsvorrichtung um die Rotationsachse auf einen bestimmten Momentenwert begrenzt werden kann.

Beim Passieren der Austragsöffnung mit dem Austragsarm wird zuvor an diesem anliegende Biomasse zur Austragsöffnung und somit weg vom Austragsarm befördert. Vor allem in einem radial äußeren Bereich des Austragsarms - also am radial äußeren Ende des Hauptarms und darüber hinaus - befindet sich dann weniger Gülle als unmittelbar vor dem Erreichen der Austragsöffnung. Im Bereich des Armfortsatzes liegt zumindest temporär keine Biomasse vor, welche noch vor dem Passieren der Austragsöffnung dafür gesorgt hat, dass der Armfortsatz weggeschwenkt wurde. Mit dem Passieren oder nach dem Passieren der Austragsöffnung sorgt das Rückstellelement dafür, dass der Armfortsatz wieder automatisch in seine Neutralstellung zurückkehrt, in welcher die jeweiligen Längsachsen des Hauptarms und des Armfortsatzes parallel zueinander ausgerichtet sind. Sodann kann wieder auf maximaler Länge des zumindest einen Austragsarms die im Beschickungssystem enthaltene Biomasse durch entsprechende Rotation der Austragsvorrichtung wiederum zur Austragsöffnung des Beschickungssystems befördert werden. Aufgrund des wegklappbaren Armfortsatzes, welcher um die parallel zur Rotationsachse verlaufende Drehachse verdrehbar am Hauptarm gelagert ist, kann ein guter Kompromiss zwischen der Austragsleistung der Austragsvorrichtung und des mittels der Antriebseinrichtung bereitzustellenden Antriebsmoments erzielt werden. Insbesondere ein Motor der Antriebseinrichtung muss nicht allzu groß dimensioniert werden, insbesondere kleiner, als wenn der zumindest eine Austragsarm der Austragsvorrichtung vollständig starr ausgebildet wäre. Das Rückstellelement sorgt neben der automatischen Rückpositionierung des Armfortsatzes in seine Neutralstellung beim oder spätestens nach dem Passieren der Austragsöffnung zudem noch dafür, dass beim Zurückschnellen des Armfortsatzes in seine Neutralstellung noch zusätzliche Biomasse die Austragsöffnung des Beschickungssystems passiert.

Eine vorteilhafte Ausführungsform der Erfindung sieht vor, dass das Rückstellelement eine Zugfeder ist, welche mit einem ersten Längsende zumindest mittelbar am Hauptarm und mit einem zweiten Längsende zumindest mittelbar am Armfortsatz angeordnet ist. Dadurch ergibt sich insbesondere der Vorteil, dass rotationsrichtungsunabhängig von der Austragsvorrichtung der Armfortsatz aus seiner Neutralstellung in zwei Richtungen um die Drehachse verdreht werden kann. Wird beispielsweise eine Drehrichtung der Antriebseinrichtung umgekehrt, so funktioniert das Rückstellelement genauso gut wie zuvor, da es nämlich unabhängig von der Verdrehrichtung des Armfortsatzes um die Drehachse das Rückstellmoment auf den Armfortsatz in Richtung seiner Neutralstellung ausübt. Mit anderen Worten wirkt das als Zugfeder ausgebildete Rückstellelement immer einer Rotation des Armfortsatzes um die Drehachse relativ zum Hauptarm ausgehend von der Neutralstellung entgegen. Insbesondere kann es vorgesehen sein, dass jeweilige Längsachsen des Hauptarms, des Armfortsatzes und des als Zugfeder ausgebildeten Rückstellelements in der Neutralstellung des Armfortsatzes miteinander fluchten. Dadurch ergibt sich auf besonders zuverlässige Weise der vorstehend beschriebene Effekt der drehrichtungsunabhängigen Rückstellwirkung des Rückstellelements. Anstelle der Zugfeder kann ganz allgemein ein Federmechanismus vorgesehen sein, z.B. in Form einer Blattfeder, Druckfeder, einem Rückstellelement aus Federstahl oder dergleichen.

Eine weitere vorteilhafte Ausführungsform der Erfindung sieht vor, dass der Armfortsatz ausgehend von der Neutralstellung um 90° um die Drehachse relativ zum Hauptarm verdrehbar ist. Vorzugsweise ist der Armfortsatz ausgehend von der Neutralstellung in zwei Richtungen bzw. Orientierungen um die Drehachse relativ zum Hauptarm um die 90° verdrehbar. Die Austragsvorrichtung kann beispielsweise jeweilige Anschläge aufweisen, welche die Verdrehung des Armfortsatzes über die 90° hinaus ausgehend von der Neutralstellung verhindern. Dadurch, dass der Armfortsatz ausgehend von der Neutralstellung um 90° um die Drehachse relativ zum Hauptarm verdrehbar ist, kann der Armfortsatz zumindest im Wesentlichen tangential zu der Kreisbahn ausgerichtet werden, welche eine Spitze des Hauptarms beschreibt, wenn die Antriebseinrichtung die Austragsvorrichtung um die Rotationsachse in Rotation versetzt. Im um 90° abgeklappten Zustand des Armfortsatzes relativ zum Hauptarm setzt der Armfortsatz der Drehbewegung bzw. Rotationsbewegung der Austragsvorrichtung zumindest so gut wie keinen Widerstand mehr entgegen, da zuvor am Armfortsatz aufgestaute Biomasse dann entsprechend der Rotation der Austragsvorrichtung entlang des abgeklappten Armfortsatzes abgleiten kann.

Gemäß einer weiteren vorteilhaften Ausführungsform der Erfindung ist es vorgesehen, dass der Hauptarm einen mittels eines Gehäuses verschlossenen Hohlraum aufweist, in welchem ein Lagerbereich des Armfortsatzes angeordnet ist, der um die Drehachse verdrehbar am Hauptarm gelagert ist. Vorzugsweise ist das Rückstellelement ebenfalls in dem Hohlraum angeordnet. Dadurch, dass der Lagerbereich des Armfortsatzes, vorzugsweise auch das Rückstellelement, in dem durch das Gehäuse verschlossenen Hohlraum angeordnet ist, kann sichergestellt werden, dass keine oder zumindest kaum Biomasse an den Lagerbereich des Armfortsatzes und insbesondere auch an das Rückstellelement gelangt. So kann dauerhaft eine besonders zuverlässige Funktionsfähigkeit der Abklappwirkung bzw. Verdrehbarkeit des Armfortsatzes sichergestellt werden. Denn keine oder zumindest so gut wie keine Biomasse kann an den Lagerbereich des Armfortsatzes und vorzugsweise auch nicht an das Rückstellelement gelangen, da der Armfortsatz und vorzugsweise auch das Rückstellelement innerhalb des durch das Gehäuse verschlossenen Hohlraums des Hauptarms angeordnet sind.

In weiterer vorteilhafter Ausgestaltung der Erfindung ist es vorgesehen, dass das Gehäuse wenigstens ein abnehmbares Gehäuseteil zum Montieren und Demontieren des Armfortsatzes aufweist. Das zumindest eine abnehmbare Gehäuseteil kann zudem auch so ausgestaltet sein, dass auch das Rückstellelement problemlos montiert und demontiert werden kann. Durch das abnehmbare Gehäuseteil ergibt sich eine Art Wartungs- und/oder Montageklappe, sodass der Armfortsatz, beispielsweise bei einem Defekt, ganz einfach ausgetauscht werden kann. Gleiches gilt auch für das Rückstellelement.

Eine weitere vorteilhafte Ausgestaltung der Erfindung sieht vor, dass der Hauptarm einen Zapfen aufweist, an welchem ein zylindrischer Bereich des Armfortsatzes um die Drehachse verdrehbar gelagert ist. Dadurch ergibt sich eine besonders zuverlässige und dauerhaft funktionierende Lagerung des Armfortsatzes am Hauptarm, und zwar verdrehbar um die besagte Drehachse.

In weiterer vorteilhafter Ausgestaltung der Erfindung ist es vorgesehen, dass zwischen dem Zapfen und dem zylindrischen Bereich des Armfortsatzes eine Buchse, insbesondere aus Polyamid, angeordnet ist. Dadurch kann die Reibung beim Verdrehen des Armfortsatzes um die Drehachse besonders gering gehalten werden. Somit bestimmt in der Hauptsache das Rückstellelement, welches das Rückstellmoment auf den Armfortsatz in Richtung der Neutralstellung ausübt, ab wann der Armfortsatz um die Drehachse wegklappt. Dadurch lässt sich das maximal mittels der Antriebseinrichtung des Beschickungssystems aufbringbare bzw. bereitstellbare Moment in Kenntnis bzw. in Abhängigkeit von den Eigenschaften des Rückstellelements definieren. Zudem sorgt die Buchse dafür, dass sowohl am Zapfen als auch am zylindrischen Bereich des Armfortsatzes ein möglichst geringer Verschleiß auftritt.

Das erfindungsgemäße Beschickungssystem zum Beschicken einer Biogasanlage mit Biomasse umfasst die erfindungsgemäße Austragsvorrichtung oder eine vorteilhafte Ausführungsform der erfindungsgemäßen Austragsvorrichtung. Des Weiteren umfasst das Beschickungssystem einen eine Austragsöffnung aufweisenden Behälter zum Aufnehmen der Biomasse, in welchem die Austragsvorrichtung um eine Rotationsachse drehbar gelagert ist. Ferner umfasst das Beschickungssystem eine mit der Schnittstelle des Anschlusskörpers der Austragsvorrichtung verbundene Antriebseinrichtung zum Antreiben der Austragsvorrichtung um die Rotationsachse zum Befördern der Biomasse zur Austragsöffnung des Behälters.

Weitere Vorteile, Merkmale und Einzelheiten der Erfindung ergeben sich aus der nachfolgenden Beschreibung eines bevorzugten Ausführungsbeispiels sowie anhand der Zeichnungen. Die vorstehend in der Beschreibung genannten Merkmale und Merkmalskombinationen sowie die nachfolgend in der Figurenbeschreibung und/oder in den Figuren alleine gezeigten Merkmale und Merkmalskombinationen sind nicht nur in der jeweils angegebenen Kombination, sondern auch in anderen Kombinationen sowie in Alleinstellung verwendbar, ohne den Rahmen der Erfindung zu verlassen.

Die Zeichnung zeigt in:
- Fig. 1: eine Perspektivansicht eines Beschickungssystem zum Beschicken einer Biogasanlage mit Biomasse, wobei das Beschickungssystem einen Behälter zum Aufnehmen der Biomasse aufweist, der einen unteren zylindrischen Teil und einen oberen trichterförmigen Teil aufweist;
- Fig. 2: eine Draufsicht auf das Beschickungssystem, wobei der trichterförmige Teil weggelassen wurde;
- Fig. 3: eine geschnittene Seitenansicht des Beschickungssystems entlang der in Fig. 2 gekennzeichneten Schnittebene A-A;
- Fig, 4: eine Perspektivansicht einer Austragsvorrichtung des Beschickungssystems, welches im Bereich eines Behälterbodens des Behälters des Beschickungssystems angeordnet werden kann, um in dem Behälter angeordnete Biomasse zu einer Austragsöffnung des Beschickungssystems zu befördern;
- Fig. 5: eine Perspektivansicht eines äußeren Bereichs eines Austragsarms der Austragsvorrichtung, wobei dieser einen verdrehbaren Armfortsatz an einem äußeren Endbereich aufweist;
- Fig. 6: eine Draufsicht auf den äußeren Bereich des Austragsarms und in
- Fig. 7: eine Längsschnittansicht des äußeren Bereichs des Austragsarms entlang der in Fig. 6 gekennzeichneten Schnittebene B-B.

In den Figuren sind gleiche oder funktionsgleiche Elemente mit den gleichen Bezugszeichen versehen worden.

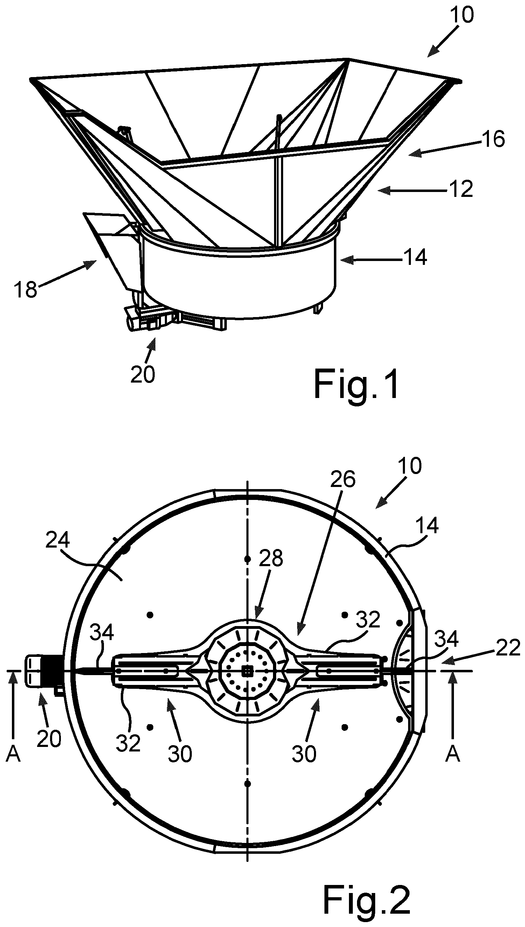

Ein Beschickungssystem 10 für eine nicht näher dargestellte Biogasanlage ist ein einer Perspektivansicht in Fig. 1 gezeigt. Das Beschickungssystem 10 dient zum Beschicken der besagten Biogasanlage mit Biomasse, beispielsweise mit Gülle, Festmist, Energiepflanzen und dergleichen. Das Beschickungssystem 10 umfasst einen Behälter 12 zum Aufnehmen der Biomasse. Der Behälter 12 umfasst einen zylindrischen Teil 14 und einen oberhalb von diesem angeordneten trichterförmigen Teil 16. Der trichterförmige Teil 16 ist nach oben hin offen, sodass beispielsweise mit einem Frontlader oder dergleichen auf einfache Weise Biomasse in den Behälter 12 eingefüllt werden kann.

Der Behälter 12 ist vorzugsweise aus Edelstahl hergestellt, sodass dieser nicht zur Rostbildung neigt, wenn feuchte Biomasse in den Behälter 12 eingebracht und darin zwischengelagert wird. Das Beschickungssystem 10 umfasst zudem ein Bedienpult 18, mittels welchem beispielsweise die Ausbringmenge des Beschickungssystems 10 reguliert werden kann. Zudem umfasst das Beschickungssystem 10 noch einen Motor 20, welcher zu einer hier nicht näher dargestellten Antriebseinrichtung des Beschickungssystems 10 gehört, mittels welcher eine hier ebenfalls nicht erkennbare Austragsvorrichtung, die in einem Bodenbereich des zylindrischen Teils 14 des Behälters 12 angeordnet ist, angetrieben werden kann.

In Fig. 2 ist das Beschickungssystem 10 in einer Draufsicht gezeigt, wobei der trichterförmige Teil 16 des Behälters 12 weggelassen wurde. Der zylindrische Teil 14 des Behälters 12 umfasst eine Austragsöffnung 22, durch welche im Behälter 12 bzw. im zylindrischen Teil 14 des Behälters 12 aufgenommene Biomasse nach außen gefördert werden kann. Im Bereich der Austragsöffnung 22 kann z.B. eine hier nicht dargestellte Förderschnecke angeordnet sein, mittels welcher die Biomasse vom Behälter 12 wegbefördert werden kann. Auf einem Boden 24 des Behälters 12 ist die zuvor erwähnte Austragsvorrichtung 26 rotierbar gelagert. Damit die Austragsvorrichtung 26 besonders reibungsarm am Boden 24 entlanggleiten kann, kann der Boden 24 beispielsweise eine Beschichtung aus Polyamid aufweisen oder auch vollständig aus Polyamid hergestellt sein.

Die Austragsvorrichtung 26 umfasst zudem einen Anschlusskörper 28 mit einer nicht näher erkennbaren Schnittstelle für die besagte Antriebseinrichtung, zu welcher unter anderem der bereits erwähnte Motor 20 gehört. Die Austragsvorrichtung 26 umfasst zudem zwei sich in radialer Richtung von dem Anschlusskörper 28 nach außen erstreckende Austragsarme 30 zum Befördern der in dem Behälter 12 angeordneten Biomasse zur Austragsöffnung 22. Die Austragsarme 30 umfassen jeweils einen starr mit dem Anschlusskörper 28 verbundenen Hauptarm 32 und einen im Bereich eines vom Anschlusskörper 28 abgewandten Längsende des Hauptarms 32 angeordneten Armfortsatz 34.

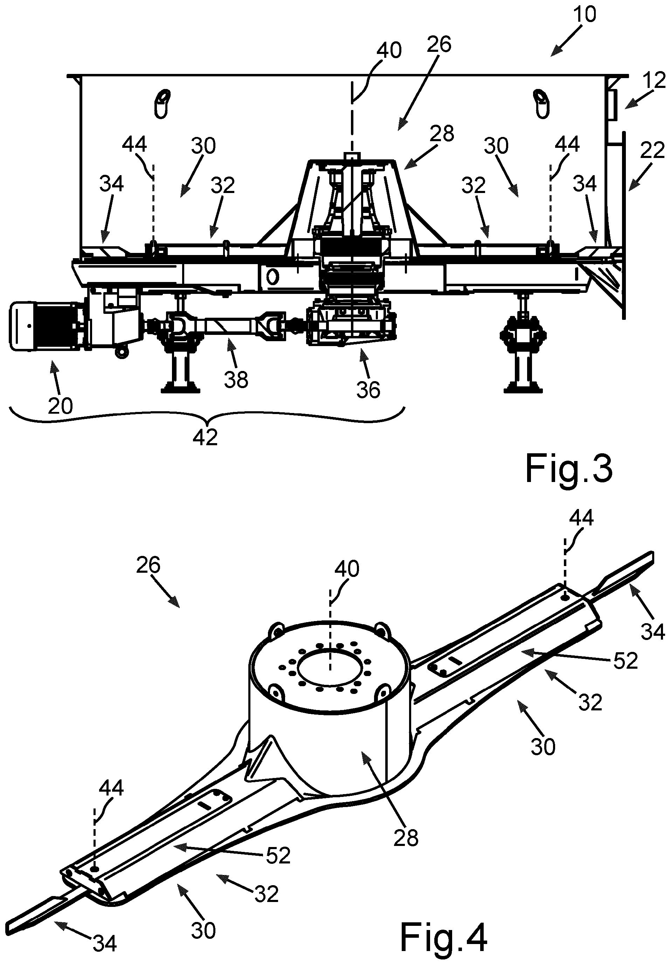

In Fig. 3 ist die Austragsvorrichtung 26 entlang der in Fig. 2 gekennzeichneten Schnittebene A-A dargestellt, wobei der trichterförmige Teil 16 des Behälters 12 wiederum weggelassen wurde. Unterseitig vom zylindrischen Teil 14 des Behälters 12 ist ein Planetengetriebe 36 angeordnet, welches mit der besagten, hier nicht näher gekennzeichneten Schnittstelle des Anschlusskörpers 28 der Austragsvorrichtung 26 verbunden ist. Das Planetengetriebe 36 wird unter Vermittlung einer Antriebswelle 38 mittels des Motors 20 angetrieben. So kann die Austragsvorrichtung 26 um eine Rotationsachse 40 der Austragsvorrichtung 26 in Rotation versetzt werden. Dadurch streichen die Austragsarme 30 über den hier nicht näher bezeichneten Boden 24 des Behälters 12 und befördern innerhalb des zylindrischen Teils 14 des Behälters 12 aufgenommene Biomasse entsprechend der Rotationsbewegung der Austragsvorrichtung 26 um die Rotationsachse 40 zur Austragsöffnung 22. Der Motor 20, die Antriebswelle 38 und das Planetengetriebe 36 bilden also die zuvor bereits erwähnte Antriebseinrichtung 42 des Beschickungssystems 10, mittels welcher die Austragsvorrichtung 26 um die Rotationsachse 40 in Rotation versetzt werden kann, um die im Behälter 12 aufgenommene Biomasse zur Austragsöffnung 22 zu befördern.

Die in radialer Richtung gesehen außen an den Hauptarmen 32 angeordneten Armfortsätze 34 sind um jeweilige parallel zur Rotationsachse 40 verlaufende Drehachsen 44 verdrehbar an den jeweiligen Hauptarmen 32 gelagert. Die Armfortsätze 34 können somit relativ zu den Hauptarmen 32 um die Drehachsen 44 verschwenkt bzw. rotiert werden.

In Fig. 4 ist die Austragsvorrichtung 26 alleine in einer Perspektivansicht gezeigt. In der vorliegenden Darstellung sind die in radialer Richtung gesehen außen angeordneten Armfortsätze 34 nochmals gut zu erkennen, welche um die besagten Drehachsen 44 verdrehbar an den Hauptarmen 32 der Austragsvorrichtung 26 angeordnet sind. Die Armfortsätze 34 sind in den Figuren 2 bis 4 jeweils in ihrer Neutralstellung gezeigt, in welcher jeweilige nicht näher bezeichnete Längsachsen der Hauptarme 32 und der Armfortsätze 34 parallel zueinander ausgerichtet sind. In dieser Neutralstellung weist die Austragsvorrichtung 26 ihre größte Spannweite auf. Die Spannweite ist dabei geringfügig kleiner als ein Innendurchmesser des zylindrischen Teils 14 des Behälters 12. In dieser Neutralstellung kämmen also die Austragsarme 30 mit ihrer maximalen Spannweite über den Boden 24 des Behälters 12 und fördern so die maximal mögliche Menge an Biomasse zur Austragsöffnung 22.

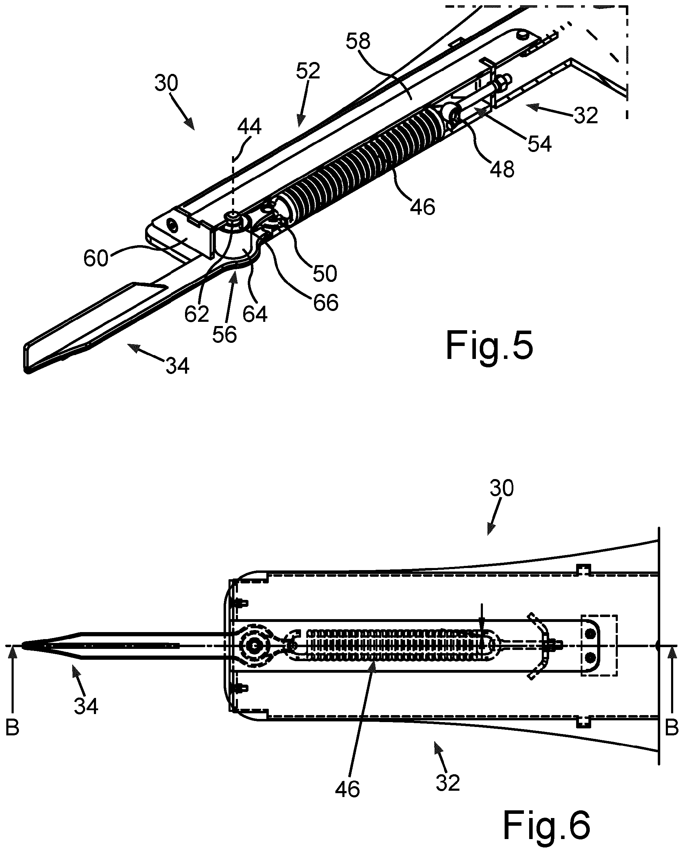

In Fig. 5 ist ein äußerer Teilbereich von einem der Austragsarme 30 in einer vergrößerten Perspektivansicht dargestellt. Je Austragsarm 30 umfasst die Austragsvorrichtung 26 ein Rückstellelement 46, welches ein Rückstellmoment auf die jeweiligen Armfortsätze 34 in Richtung der besagten Neutralstellung ausübt, in welcher jeweilige Längsachsen des betreffenden Hauptarms 32 und des betreffenden Armfortsatzes 34 parallel zueinander ausgerichtet sind. Im vorliegend gezeigten Fall handelt es sich bei den Rückstellelementen 46 jeweils um eine Zugfeder, welche jeweils mit einem ersten Längsende 48 am betreffenden Hauptarm 32 und mit einem zweiten Längsende 50 am betreffenden Armfortsatz 34 angeordnet ist. In der hier gezeigten Neutralstellung des Armfortsatzes 34 fluchtet eine Längsachse des Rückstellelements 46 mit der Längsachse des Armfortsatzes.

Die Armfortsätze 34 sind derart um die jeweilige Drehachse 44 verdrehbar gelagert, dass die Armfortsätze 34 ausgehend von der hier gezeigten Neutralstellung um 90° um die Drehachse 44 relativ zum jeweiligen Hauptarm 32 verdreht werden können. Die Verdrehung um die Drehachse 44 kann dabei gleichermaßen rechtsherum als auch linksherum um die Drehachse 44 erfolgen. Dadurch, dass es sich bei den Rückstellelementen 46 um jeweilige Zugfedern handelt, wird drehrichtungsunabhängig um die Drehachse 44 betragsmäßig immer das gleiche Rückstellmoment auf die jeweiligen Armfortsätze 34 in Richtung ihrer Neutralstellung ausgeübt.

Die beiden Hauptarme 32 weisen einen jeweiligen mittels eines Gehäuses 52 verschlossenen Hohlraum 54 auf, in welchem ein Lagerbereich 56 der jeweiligen Armfortsätze 34 angeordnet ist, der um die jeweilige Drehachse 44 verdrehbar am jeweiligen Hauptarm 32 gelagert ist. Wie zu erkennen, befinden sich die als Zugfedern ausgebildeten Rückstellelemente 46 ebenfalls in den besagten Hohlräumen 54, welche von jeweiligen Gehäusen 52 umgeben sind.

Die Gehäuse 52, welche die jeweiligen Hohlräume 54 umschließen, weisen jeweils zwei abnehmbare Gehäuseteile 58, 60 auf. Diese können beispielsweise durch Schraubverbindungen wieder lösbar mit dem restlichen Gehäuse 52 verbunden sein. So können bei demontierten Gehäuseteilen 58, 60 die Armfortsätze 34 mit ihren Lagerbereichen 56 sowie die jeweiligen als Zugfedern ausgebildeten Rückstellelemente 46 in den jeweiligen Hohlräumen 54 der Hauptarme 32 angeordnet werden. Anschließend können die Gehäuse 52 durch entsprechende Montage der Gehäuseteile 58, 60 verschlossen werden, sodass in die Hohlräume 54 zumindest im Wesentlichen keine Biomasse eindringen kann.

Die jeweiligen Hauptarme 32 weisen im Bereich ihrer jeweiligen Längsenden, welche von dem hier nicht erkennbaren Anschlusskörper 28 abgewandt sind, jeweilige Zapfen 62 auf, an welchen jeweilige zylindrische Bereiche 64 der Armfortsätze 34 um die jeweiligen Drehachsen 44 verdrehbar gelagert sind. Die zylindrischen Bereichen 64 sind dabei jeweils ein Bestandteil der jeweiligen Lagerbereiche 56 der Armfortsätze 34. Zwischen den jeweiligen Zapfen 62 und den zylindrischen Bereichen 64 der Armfortsätze 34 ist jeweils eine Buchse 66, vorzugsweise aus Polyamid, angeordnet. Die Buchse 66 minimiert die Reibung zwischen den Zapfen 62 und den zylindrischen Bereichen 64, wenn die Armfortsätze 34 um die jeweiligen Drehachsen 44 relativ zu den Hauptarmen 32 verschwenkt bzw. verdreht werden.

In Fig. 6 ist einer der beiden Austragsarme 30 der Austragsvorrichtung 26 in einer teiltransparenten Draufsicht gezeigt. Sobald aufgrund einer entsprechend angestauten Menge an Biomasse an den Armfortsätzen 34 ein Moment auf diese Armfortsätze 34 um die jeweiligen Drehachsen 44 wirkt, welches größer als das momentan von den Rückstellelementen 46 ausgeübte Rückstellmoment ist, werden die Armfortsätze 34 um die Drehachsen 44 relativ zu den Hauptarmen 32 verschwenkt. Gemäß der vorliegenden Darstellung werden die Armfortsätze 34 also je nach Drehrichtung der Austragsvorrichtung 26 entweder gegen den Uhrzeigesinn oder mit dem Uhrzeigersinn um die hier nicht näher gekennzeichneten Drehachsen 44 verschwenkt. Dies kann so weit geschehen, bis die Armfortsätze 34 quer zu den Hauptarmen 32 angeordnet sind. In dieser Endstellung verlaufen die Längsachsen der Armfortsätze 34 also quer zu den Längsachsen der Hauptarme 32. Die Armfortsätze 34 weisen also um die Drehachse 44 insgesamt einen Rotationsfreiheitsgrad von 180° auf. Sobald die Armfortsätze 34 aus ihrer Neutralstellung heraus um die jeweiligen Drehachsen 44 verschwenkt werden, werden die als Zugfedern ausgebildeten Rückstellelemente 46 gedehnt und entsprechend dieser Dehnung üben diese das besagte Rückstellmoment auf die Armfortsätze 34 auf.

Sobald die jeweiligen Armfortsätze 34 die Austragsöffnung 22 des Behälters 12 passieren, gelangt an den Austragsarmen 30 angestaute Biomasse durch die Austragsöffnung 22 aus dem Inneren des Behälters 12 heraus. Zuvor auch noch an den Armfortsätzen 34 aufgestaute Biomasse wird also im Bereich der Armfortsätze 34 entfernt, in Folge dessen die Biomasse keine Kraft mehr auf die Armfortsätze 34 ausübt, welche sie von der Neutralstellung weggebogen bzw. verdreht gehalten hatte. Die Rückstellelemente 46 sorgen dann beim jeweiligen Passieren der Austragsöffnung 22 dafür, dass die zuvor gegenüber den Hauptarmen 32 verschwenkten Armfortsätze 34 wieder zurück in ihre Neutralstellung gelangen.

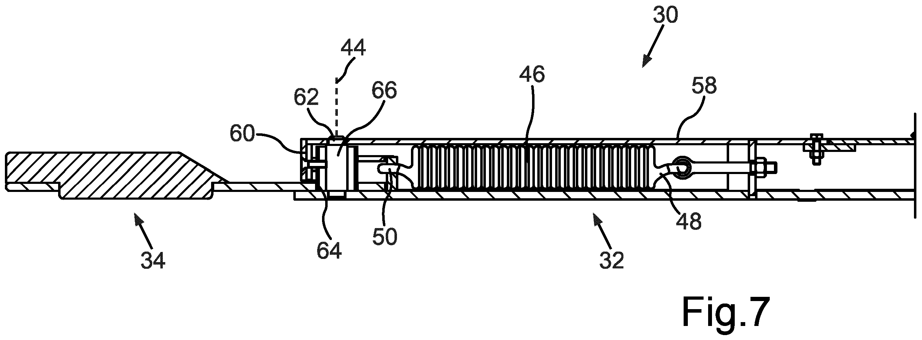

In Fig. 7 ist einer der beiden Austragsarme 30 in einer Längsschnittdarstellung entlang der in Fig. 6 gekennzeichneten Schnittebene B-B gezeigt. In der vorliegenden Darstellung ist nochmals gut zu erkennen, wie die Armfortsätze 34 an den Hauptarmen 32 gelagert sind. Die Anordnung der Rückstellelemente 46 ist hier ebenfalls nochmals gut zu erkennen. Durch die beschriebene drehbare Lagerung und Anordnung der Armfortsätze 34 kann sichergestellt werden, dass beim Rotieren der Austragsvorrichtung 26 um die Rotationsachse 40 bei einer entsprechenden Krafteinwirkung in Umfangsrichtung - bezogen auf die Rotationsachse 40 - die Armfortsätze 34 um die jeweiligen Drehachsen 44 verschwenkt werden bzw. wegklappen. Staut sich also genügende Biomasse an den Armfortsätzen 34 an, so klappen diese um die Drehachsen 44 weg, maximal so weit, bis die Armfortsätze 34 in einem rechten Winkel zu den Hauptarmen 32 angeordnet sind. In dieser Endstellung üben die Hauptarme 32 ihr geringstes Gegenmoment aus, welches entgegen der Rotationsrichtung der Austragsarme 30, also entgegen des vom Motor 20 aufgebrachten Antriebsmoments wirkt.

Die Rückstellelemente 46 sind vorzugsweise so ausgebildet bzw. eingestellt, dass die Armfortsätze 34 so rechtzeitig um die Drehachsen 44 wegklappen, dass das der Rotationsbewegung der Austragsvorrichtung 26 entgegenwirkende Moment nicht so weit ansteigen kann, dass das maximal vom Motor 20 bereitstellbare Moment überstiegen wird. Die Dimensionierung des Motors 20 kann somit kleiner erfolgen als wenn die Austragsarme 30 über ihre gesamte Länge starr ausgebildet wären. Durch die wegklappbaren Armfortsätze 34 kann somit ein guter Kompromiss zwischen einer Austragsleistung der Austragsvorrichtung 26 hinsichtlich der Biomasse und dem zur Rotation der Austragsvorrichtung 26 aufzubringendem Antriebsmoment erzielt werden.

### BEZUGSZEICHENLISTE:

- 10: Beschickungssystem
- 12: Behälter
- 14: zylindrischer Teil des Behälters
- 16: trichterförmiger Teil des Behälters
- 18: Bedienpult des Beschickungssystems
- 20: Motor des Beschickungssystems
- 22: Austragsöffnung des Beschickungssystems
- 24: Boden des Behälters
- 26: Austragsvorrichtung
- 28: Anschlusskörper der Austragsvorrichtung
- 30: Austragsarme der Austragsvorrichtung
- 32: Hauptarme der Austragsarme
- 34: Armfortsätze der Austragsarme
- 36: Planetengetriebe des Beschickungssystems
- 38: Antriebswelle des Beschickungssystems
- 40: Rotationsachse der Austragsvorrichtung
- 42: Antriebseinrichtung des Beschickungssystems
- 44: Drehachsen der Armfortsätze
- 46: Rückstellelemente der Austragsvorrichtung
- 48: erste Längsenden der Rückstellelemente
- 50: zweite Längsenden der Rückstellelemente
- 52: Gehäuse der Hauptarme
- 54: Hohlräume der Hauptarme
- 56: Lagerbereiche der Armfortsätze
- 58: Gehäuseteile der Gehäuse
- 60: Gehäuseteile der Gehäuse
- 62: Zapfen der Hauptarme
- 64: zylindrische Bereiche der Armfortsätze
- 66: Buchsen der Armfortsätze

## Patentansprüche

1. Austragsvorrichtung (26) für ein Beschickungssystem (10), das zum Beschicken einer Biogasanlage mit Biomasse ausgelegt ist, umfassend
- einen Anschlusskörper (28) mit einer Schnittstelle für eine Antriebseinrichtung (42) des Beschickungssystems (10), mittels welcher die Austragsvorrichtung (26) um eine Rotationsachse (40) in Rotation versetzbar ist;
- zumindest einen sich in radialer Richtung von dem Anschlusskörper (28) nach außen erstreckenden Austragsarm (30) zum Befördern der Biomasse zu einer Austragsöffnung (22) des Beschickungssystems (10), wobei der Austragsarm (30) einen starr mit dem Anschlusskörper (28) verbundenen Hauptarm (32) und einen im Bereich eines vom Anschlusskörper (28) abgewandten Längsende des Hauptarms (32) angeordneten Armfortsatz (34) aufweist, der um eine parallel zur Rotationsachse (40) verlaufende Drehachse (44) verdrehbar am Hauptarm (32) gelagert ist;
- ein Rückstellelement (46), welches ein Rückstellmoment auf den Armfortsatz (34) in Richtung einer Neutralstellung ausübt, in welcher jeweilige Längsachsen des Hauptarms (32) und des Armfortsatzes (34) parallel zueinander ausgerichtet sind.

2. Austragsvorrichtung (26) nach Anspruch 1,
**dadurch gekennzeichnet, dass**
das Rückstellelement (46) eine Zugfeder ist, welche mit einem ersten Längsende (48) zumindest mittelbar am Hauptarm (32) und mit einem zweiten Längsende (50) zumindest mittelbar am Armfortsatz (34) angeordnet ist.

3. Austragsvorrichtung (26) nach einem der vorhergehenden Ansprüche,
**dadurch gekennzeichnet, dass**
der Armfortsatz (34) ausgehend von der Neutralstellung um 90° um die Drehachse (44) relativ zum Hauptarm (32) verdrehbar ist.

4. Austragsvorrichtung (26) nach einem der vorhergehenden Ansprüche,
**dadurch gekennzeichnet, dass**
der Hauptarm (32) einen mittels eines Gehäuses (52) verschlossenen Hohlraum (54) aufweist, in welchem ein Lagerbereich (56) des Armfortsatzes (34) angeordnet ist, der um die Drehachse (44) verdrehbar am Hauptarm (34) gelagert ist.

5. Austragsvorrichtung (26) nach Anspruch 4,
**dadurch gekennzeichnet, dass**
das Rückstellelement (46) ebenfalls in dem Hohlraum (54) angeordnet ist.

6. Austragsvorrichtung (26) nach einem der Ansprüche 4 oder 5,
**dadurch gekennzeichnet, dass**
das Gehäuse (52) wenigstens ein abnehmbares Gehäuseteil (58, 60) zum Montieren und Demontieren des Armfortsatzes (34) aufweist.

7. Austragsvorrichtung (26) nach einem der vorhergehenden Ansprüche,
**dadurch gekennzeichnet, dass**
der Hauptarm (32) einen Zapfen (62) aufweist, an welchem ein zylindrischer Bereich (64) des Armfortsatzes (34) um die Drehachse (44) verdrehbar gelagert ist.

8. Austragsvorrichtung (26) nach Anspruch 7,
**dadurch gekennzeichnet, dass**
zwischen dem Zapfen (62) und dem zylindrischen Bereich (64) des Armfortsatzes (34) eine Buchse (66), insbesondere aus Polyamid, angeordnet ist.

9. Beschickungssystem (10) zum Beschicken einer Biogasanlage mit Biomasse, umfassend
- eine Austragsvorrichtung (26) nach einem der vorhergehenden Ansprüche;
- einen eine Austragsöffnung (22) aufweisenden Behälter (12) zum Aufnehmen der Biomasse, in welchem die Austragsvorrichtung (26) um eine Rotationsachse (40) drehbar gelagert ist;
- eine mit der Schnittstelle des Anschlusskörpers (28) der Austragsvorrichtung (26) verbundene Antriebseinrichtung (42) zum Antreiben der Austragsvorrichtung (26) um die Rotationsachse (40) zum Befördern der Biomasse zur Austragsöffnung (22) des Behälters (12).

## Claims

1. Delivery device (26) for a charging system (10), which is configured for charging a biogas system with biomass, comprising
- a connecting body (28) with an interface for a drive device (42) of the charging system (10), by means of which the delivery device (26) can be set into rotation about an axis of rotation (40);
- at least one discharge arm (30) extending outwards in radial direction from the connecting body (28) for conveying the biomass to a discharge opening (22) of the charging system (10), wherein the discharge arm (30) has a main arm (32) connected rigidly to the connecting body (28) and an arm extension (34) arranged in the region of a longitudinal end of the main arm (32) facing away from the connecting body (28), which arm extension is mounted on the main arm (32) so as to be rotatable about an axis of rotation (44) extending parallel to the axis of rotation (40);
- a restoring element (46), which exerts a restoring moment on the arm extension (34) in the direction of a neutral position, in which respective longitudinal axes of the main arm (32) and the arm extension (34) are aligned parallel to one another.

2. Delivery device (26) according to claim 1,
**characterised in that**
the restoring element (46) is a tension spring, which is arranged with a first longitudinal end (48) at least indirectly on the main arm (32) and with a second longitudinal end (50) at least indirectly on the arm extension (34).

3. Delivery device (26) according to any of the preceding claims,
**characterised in that**
the arm extension (34), starting from the neutral position, is rotatable by 90° about the axis of rotation (44) relative to the main arm (32).

4. Delivery device (26) according to any of the preceding claims,
**characterised in that**
the main arm (32) has a cavity (54) which is closed by means of a housing (52) in which a bearing region (56) of the arm extension (34) is arranged, which is mounted on the main arm (34) so as to be rotatable about the axis of rotation (44).

5. Delivery device (26) according to claim 4,
**characterised in that**
the restoring element (46) is also arranged in the cavity (54).

6. Delivery device (26) according to any of claims 4 or 5,
**characterised in that**
the housing (52) has at least one removable housing part (58, 60) for mounting and removing the arm extension (34).

7. Delivery device (26) according to any of the preceding claims,
**characterised in that**
the main arm (32) has a pin (62), on which a cylindrical region (64) of the arm extension (34) is rotatably mounted about the axis of rotation (44).

8. Delivery device (26) according to claim 7,
**characterised in that**
a bushing (66), in particular made of polyamide, is arranged between the pin (62) and the cylindrical region (64) of the arm extension (34).

9. Charging system (10) for charging a biogas system with biomass, comprising
- a delivery device (26) according to any of the preceding claims;
- a container (12) having an discharge opening (22) for receiving the biomass, in which the delivery device (26) is mounted rotatably about an axis of rotation (40);
- a drive device (42) connected to the interface of the connecting body (28) of the delivery device (26) for driving the delivery device (26) about the axis of rotation (40) for conveying the biomass to the discharge opening (22) of the container (12).

## Revendications

1. Dispositif de décharge (26) pour un système de chargement (10) qui est conçu pour charger une installation de biogaz en biomasse, comprenant
- un corps de raccord (28) avec une interface pour un dispositif d'entraînement (42) du système de chargement (10), au moyen duquel le dispositif de décharge (26) peut être mis en rotation autour d'un axe de rotation (40) ;
- au moins un bras de décharge (30) s'étendant radialement vers l'extérieur à partir du corps de raccord (28) pour transporter la biomasse vers une ouverture de décharge (22) du système de chargement (10), dans lequel le bras de décharge (30) présente un bras principal (32) connecté de manière rigide au corps de raccord (28) et une extension de bras (34) disposée au niveau d'une extrémité longitudinale du bras principal (32) opposée au corps de raccord (28), qui est montée sur le bras principal (32) de manière à pouvoir tourner autour d'un axe de pivot (44) s'étendant parallèlement à l'axe de rotation (40) ;
- un élément de rappel (46), lequel exerce un couple de rappel sur l'extension de bras (34) en direction d'une position neutre dans laquelle les axes longitudinaux respectifs du bras principal (32) et de l'extension de bras (34) sont alignés parallèlement l'un à l'autre.

2. Dispositif de décharge (26) selon la revendication 1,
**caractérisé en ce que**
l'élément de rappel (46) est un ressort de traction, lequel est disposé avec une première extrémité longitudinale (48) au moins indirectement sur le bras principal (32) et avec une seconde extrémité longitudinale (50) au moins indirectement sur l'extension de bras (34).

3. Dispositif de décharge (26) selon l'une quelconque des revendications précédentes,
**caractérisé en ce que**
l'extension de bras (34) partant de la position neutre peut tourner de 90° autour de l'axe de pivot (44) par rapport au bras principal (32).

4. Dispositif de décharge (26) selon l'une quelconque des revendications précédentes,
**caractérisé en ce que**
le bras principal (32) présente une cavité (54) fermée au moyen d'un boîtier (52) dans laquelle une section de palier (56) de l'extension de bras (34) est disposée, qui est montée sur le bras principal (34) de manière à pouvoir tourner autour de l'axe de pivot (44).

5. Dispositif de décharge (26) selon la revendication 4,
**caractérisé en ce que**
l'élément de retour (46) est également disposé dans la cavité (54).

6. Dispositif de décharge (26) selon l'une quelconque des revendications 4 ou 5,
**caractérisé en ce que**
le boîtier (52) présente au moins une partie de boîtier amovible (58, 60) pour monter et démonter l'extension de bras (34).

7. Dispositif de décharge (26) selon l'une quelconque des revendications précédentes,
**caractérisé en ce que**
le bras principal (32) présente un tourillon (62) sur lequel une section cylindrique (64) de l'extension de bras (34) est montée de manière rotative autour de l'axe de pivot (44).

8. Dispositif de décharge (26) selon la revendication 7,
**caractérisé en ce**
**qu'**une douille (66), en particulier en polyamide, est disposée entre le tourillon (62) et la section cylindrique (64) de l'extension de bras (34).

9. Système de chargement (10) pour charger une installation de biogaz en biomasse, comprenant
- un dispositif de décharge (26) selon l'une quelconque des revendications précédentes ;
- un conteneur (12) présentant une ouverture de décharge (22) pour recevoir la biomasse, dans lequel le dispositif de décharge (26) est monté de manière rotative autour d'un axe de rotation (40) ;
- un dispositif d'entraînement (42) connecté à l'interface du corps de raccord (28) du dispositif de décharge (26) pour entraîner le dispositif de décharge (26) autour de l'axe de rotation (40) pour transporter la biomasse vers l'ouverture de décharge (22) du conteneur (12).
